# EUROPÄISCHE PATENTANMELDUNG

(11) **EP 2 388 029 A1**
(43) Veröffentlichungstag der Anmeldung: **23.11.2011**
(21) Anmeldenummer: 10075202.1
(22) Anmeldetag: 17.05.2010
(51) Int. Cl.: A61M 1/12, F04F 5/02

(54) **Pumpenanordnung**

(71) Anmelder: ECP Entwicklungsgesellschaft mbH, 12247 Berlin (DE)
(72) Erfinder: Liebing, Rainer, 14469 Potsdam (DE)
(74) Vertreter: Pfenning, Meinig & Partner GbR

(57) **Zusammenfassung**

Gegenstand der vorliegenden Erfindung ist eine Pumpenanordnung (1, 10, 20, 30, 40, 50), insbesondere zum Einsatz in körpereigenen Gefäßen, mit einer Pumpe (11, 41, 51) und einer die Pumpe aufnehmenden und einen Strömungskanal (S) begrenzenden Umhüllung (12, 42, 52) mit einer distalen Ansaugöffnung (13, 43, 53) und einer proximalen Ausströmöffnung (14, 29, 39, 44, 54) zur Erzeugung eines Treibstroms mittels der Pumpe, wobei die Pumpe in einem ersten fluiddichten und die distale Ansaugöffnung aufweisenden Abschnitt (12a, 42a, 52a) angeordnet ist und ein zweiter fluiddichter Abschnitt (12b, 42b, 52b) die proximale Ausströmöffnung umfasst.

Erfindungsgemäß befindet sich zwischen dem ersten und zweiten Abschnitt eine weitere zwischen der Ansaugöffnung und Ausströmöffnung gelegene Einlassöffnung (15), wobei der erste Abschnitt und der zweite Abschnitt derart zueinander angeordnet sind, dass die Einlassöffnung proximal der Pumpe in den Strömungskanal mündet.

## Beschreibung

Die Erfindung liegt auf dem Gebiet des Maschinenbaus und der Feinmechanik und ist insbesondere im medizinischen Bereich mit Vorteil einsetzbar.

Gegenstand ist dabei eine Pumpenanordnung nach dem Oberbegriff des Anspruchs 1.

Aus dem Stand der Technik sind zunehmend Pumpenanordnungen insbesondere zum Einsatz in körpereigenen Gefäßen bekannt. Diese können beispielsweise zur kurzfristigen Herzunterstützung eingesetzt werden, um den Herzmuskel eines Patienten nach einem kardiogenen Schock (Myokardinfarkt) zu entlasten. Hierbei wird bisweilen auf transfemoral implantierte Mikroaxialpumpen zurückgegriffen.

Eine derartige Pumpenanordnung ist beispielsweise aus der EP 2 047 872 A1 bekannt. Die dort offenbarte Pumpenanordnung umfasst eine Pumpe, eine die Pumpe aufnehmende Umhüllung mit einer distalen Ansaugöffnung und einer proximalen Ausströmöffnung, wobei die Pumpe im Betrieb einen Treibstrom von der distalen Ansaugöffnung zur proximalen Ausströmöffnung hin erzeugt. Zwischen der Ansaugöffnung und der Ausströmöffnung erstreckt sich somit ein Strömungskanal. Die Pumpe ist dabei in einem ersten fluiddichten und die distale Ansaugöffnung aufweisenden Abschnitt der Umhüllung, welcher als PU-Bespannung eines Gehäuses ausgebildet ist, angeordnet. Weiterhin ist ein zweiter fluiddichter Abschnitt der Umhüllung vorhanden, welcher die proximale Ausströmöffnung umfasst und als Abströmschlauch ausgebildet ist. Der Abströmschlauch wird stoffschlüssig mit der PU-Bespannung verbunden. Die Pumpenanordnung ist derart angeordnet, dass die als Rotor ausgebildete Pumpe beispielsweise in einer Herzkammer angeordnet werden kann, wobei sich der Abströmschlauch von der Herzkammer in die Aorta hinein erstreckt.

Sämtliches über Ausströmöffnungen des Abströmschlauchs in die Aorta tretende Blut gelangt durch die Ansaugöffnung in den durch die Umhüllung gebildeten Strömungskanal und passiert dabei den Rotor. Mit anderen Worten: Der durch die Pumpe geförderte Förderstrom ist identisch mit dem an der Ausströmöffnung austretenden Gesamtstrom.

Auch die Gegenstände der Druckschriften DE 41 24 299 A1, DE 10 2004 054 714 A1, WO 2007/112033 A2 und US 2008/132748 A1 verfahren nach dem vorhergehend benannten Prinzip.

Da sämtliches Blut in direkten Kontakt mit der Pumpe kommt, ist bei der Herstellung der Pumpen ein besonders hoher Aufwand nötig, um die blutschädigende Wirkung der beweglichen Pumpenteile zu reduzieren. Diese blutschädigende Wirkung manifestiert sich in direkter mechanischer Scherung an beweglichen und stationären Pumpenteilen und in Scherung durch auftretende Schubspannungsfelder im Fluid während der Passage des Strömungskanals (s. o.). Daher entstehen auch Pumpengeometrien, welche nicht mittels eines minimalinvasiven Eingriffs eingesetzt werden können.

Der vorliegenden Erfindung liegt die Aufgabe zugrunde, die Gefahr einer Schädigung des Blutes bzw. des durch die Pumpe transportierten Fluids zu verringern. Besonderes Augenmerk bei der medizinischen Verwendung der nachstehenden Pumpenanordung(en) ist auf die Wahl der Werkstoffe zu legen. Diese sollten sinnhafterweise den Ansprüchen an die Biokompatibilität genügen. Interaktionen mit Fremdkörperoberflächen bilden ergänzend zu obiger Ausführung den dritten großen Aspekt bei der Blutschädigung und werden seit geraumer Zeit hinreichend untersucht.

Erfindungsgemäß wird zwischen dem ersten und dem zweiten Abschnitt eine weitere Einlassöffnung eingebracht, wobei der erste Abschnitt und der zweite Abschnitt derart zueinander angeordnet sind, dass die Einlassöffnung proximal der Pumpe in den Strömungskanal mündet. Hierbei kann die weitere Einlassöffnung auch als Einlasskanal ausgebildet sein, der distal bzw. proximal der Pumpe oder auch auf Höhe der Pumpe seine Einlassöffnung hat, der aber erst proximal der Pumpe in den Strömungskanal mündet.

Mithilfe des erfindungsgemäßen Merkmals ist es möglich, dass ein die Pumpe durchlaufender Treibstrom durch den Strömungskanal an der Mündung der Einlassöffnung in Richtung der Auslassöffnung vorbeiströmt und somit gegenüber dem in der Einlassöffnung vorliegenden Fluiddruck einen Druckabfall bewirkt, welcher zu einem Nachsaugen von Fluid durch die Einlassöffnung und in den Strömungskanal führt. Hierdurch ist der an der Ausströmöffnung ausströmende Gesamtstrom größer als der die Pumpe direkt passierende und von dieser geförderte Treibstrom, da durch die Einlassöffnung ein zusätzlicher Saugstrom hinzukommt.

Der Saugstrom entsteht dadurch, dass durch den Treibstrom eine Sogwirkung entsteht, wie sie auch bei einigen Turbinenarten oder Wasserstrahlpumpen auftritt. Bei der Sogwirkung wird ein Impuls durch Reibung oder Viskosität oder turbulente Mischung des Fluids vom Treibstrom auf den Saugstrom übertragen. Auf diese Weise entstehen viskose, turbulente Schubspannungen. Die Impulsrichtung des Treibmittelstroms wird auf Teilchen aus dem zu fördernden Medium des Saugstroms übertragen, welche in eine Zone stromabwärts innerhalb der Umhüllung transportiert werden.

Im Wesentlichen wird mithilfe des erfindungsgemäßen Merkmals das Prinzip einer Strahlpumpe umgesetzt, wobei der die Pumpe direkt passierende und von dieser geförderte Treibstrom einen durch die weitere Einlassöffnung eintretenden Saugstrom mit sich reißt.

Die Einlassöffnung erstreckt sich dabei zwischen dem ersten und zweiten Abschnitt, wobei der Eingang zur Einlassöffnung proximal oder distal der Pumpe liegen kann, wobei es vorteilhaft ist, wenn der Eingang distal der Ausströmöffnung liegt. Lediglich die Mündung der Einlassöffnung in den Strömungskanal sollte proximal der Pumpe liegen, so dass der Saugeffekt, welcher durch den Treibstrom verursacht wird, gut genutzt wird.

Besonders vorteilhaft ist eine zwischen der Ansaugöffnung und der Ausströmöffnung gebildete Einlassöffnung.

Der erste und zweite Abschnitt der Umhüllung können einstückig oder als voneinander getrennte Bauteile ausgebildet sein.

In einer ersten Ausführungsform ist der Querschnitt des proximalen Endes des ersten Abschnitts kleiner als der Querschnitt des distalen Endes des zweiten Abschnitts. Hierdurch wird der Förderstrom auf eine Fläche des Querschnitts des proximalen Endes des ersten Abschnitts konzentriert und kann beim Eintritt in den zweiten Abschnitt weiteres Medium mit sich reißen, welches zumindest durch Zugänge im Bereich der verbleibenden Fläche des Querschnitts des distalen Endes des zweiten Abschnitts einfließen kann.

In einer weiteren Ausführungsform verjüngt sich der Querschnitt des ersten Abschnitts zu seinem proximalen Ende hin. Durch die Verjüngung ist der erste Abschnitt an seinem proximalen Ende düsenförmig ausgebildet. Dies führt zu einer Verbesserung des Wirkungsgrades und somit zu einer Erhöhung des angesogenen Saugstromes. Zudem hilft das Merkmal, eine Verkleinerung der gesamten Pumpenanordnung zu bewirken.

In einer weiteren Ausführungsform überlappen sich das distale Ende des zweiten Abschnitts und das proximale Ende des ersten Abschnitts, d. h., das distale Ende des zweiten Abschnitts liegt distaler als das proximale Ende des ersten Abschnitts. Dabei ist es vorteilhaft, wenn die Einlassöffnung zwischen dem ersten und dem zweiten Abschnitt jeweils vom distalen Ende des zweiten Abschnitts zum proximalen Ende des ersten Abschnitts hin als Ansaugkanal oder kanalartig ausgebildet ist. Hierdurch strömt der Saugstrom durch den Ansaugkanal vorzugsweise nahezu koaxial zur Förderrichtung des Treibstroms in den Strömungskanal in Richtung der Ausströmöffnung ein. Hierbei wird dem Saugstrom aufgrund der vorzugsweise in Richtung des Förderstroms gerichteten Hauptachse des Ansaugkanals bereits ein Impuls des Treibstroms in Richtung der Ausströmöffnung übertragen. Dies führt zu einer Verbesserung des Wirkungsgrads.

In einer weiteren Ausführungsform befindet sich das distale Ende des zweiten Abschnitts proximaler als oder auf gleicher Höhe wie das proximale Ende des ersten Abschnitts. Durch die Beabstandung trifft der konzentrierte, aus dem proximalen Ende des ersten Abschnitts austretende Treibstrom auf ein Fluid unterschiedlichen Drucks und unterschiedlicher Richtungen, so dass der konzentrierte Förderstrom einem Fluid höherer Dichte gleich weiter in den durch das distale Ende des zweiten Abschnitts definierten Beginn des zweiten Abschnitts hineinfließt und Fluid, welches sich zwischen dem ersten und dem zweiten Abschnitt befindet, mit sich zieht. Hierdurch wird der Gesamtstrom gegenüber dem durch die Pumpe tretenden Treibstrom größer. Hierbei ist jedoch darauf zu achten, dass die Beabstandung zwischen dem distalen Ende des zweiten Abschnitts und dem proximalen Ende des ersten Abschnitts gering gehalten wird, um keine Zerstreuung des Förderstroms gegenüber dem außerhalb der Umhüllung vorliegenden Strom zu bewirken. Der Abstand sollte näherungsweise von 0 bis 1/4 des Durchmessers der proximalen Austrittsöffnung des ersten Abschnitts betragen.

In einer weiteren Ausführungsform umfasst der zweite Abschnitt zumindest einen Teilbereich aus einem flexiblen Material. Hierdurch ist es möglich, dass ein zwischen beispielsweise einer Herzkammer und einem Blutgefäß, wobei die Herzkammer und das Blutgefäß mittels einer sich rhythmisch öffnenden und schließenden Klappe verbunden sind, liegender zweiter Abschnitt durch die Klappe eingedrückt werden kann und so das Fluid im Einklang mit der rhythmischen Bewegung der Klappe befördert wird. Als geeignete Materialien sind beispielsweise PU, PE, PP, Silikon oder Parilene geeignet, sofern sie die mechanischen und geometrischen Anforderungen sowie die Anforderungen an Biokompatibilität erfüllen.

In einer weiteren Ausführungsform weist die Pumpenanordnung ein die Pumpe aufnehmendes Gehäuse auf. Dies ist insbesondere dann geeignet, wenn es sich bei der Pumpe um eine komprimierbare Pumpe handelt, welche mittels eines Katheters zusammen mit dem Gehäuse an ihren Arbeitsort befördert wird. Zudem verleiht das Gehäuse der Pumpenanordnung eine zusätzliche Stabilität. Das Gehäuse kann beispielsweise aus Nitinol hergestellt werden.

Beim Vorhandensein eines Gehäuses kann der erste Abschnitt als eine Ummantelung des Gehäuses oder eine Beschichtung des Gehäuses ausgebildet sein, wobei lediglich ein Teilbereich des Gehäuses, vorzugsweise ein axialer Teilbereich, fluiddicht ummantelt bzw. beschichtet sein muss. Als Beschichtungen bzw. Ummantelungen eignen sich hier Materialien, wie sie bereits bei der Beschreibung des zweiten Abschnitts aus einem flexiblen Material aufgeführt wurden.

In einer weiteren Ausführungsform weist das Gehäuse proximal der Pumpe eine Einschnürung und/oder eine Ausbauchung auf. Hierbei ist unter einer Einschnürung eine Verjüngung des Querschnitts des Gehäuses gegenüber dem die Pumpe aufnehmenden Bereich des Gehäuses zu verstehen. Eine Ausbauchung weist einen gegenüber einer Einschnürung oder gegenüber dem die Pumpe aufnehmenden Bereich vergrößerten Querschnitt des Gehäuses auf. Mithilfe einer derartigen Formgebung können Verjüngungen des ersten Abschnitts bzw. größere Querschnitte des zweiten Abschnitts besonders leicht und vorteilhaft realisiert werden. Auch der zweite Abschnitt kann mit dem Gehäuse verbunden sein.

In einer weiteren Ausführungsform sind der erste und der zweite Abschnitt stoffschlüssig miteinander verbunden oder vorzugsweise einstückig ausgebildet.

In einer weiteren Ausführungsform ist der zweite Abschnitt als ein Abströmschlauch ausgebildet.

Weiterhin kann der zweite Abschnitt im Bereich der Einlassöffnung einen Stützring aufweisen, welcher eine Beabstandung des ersten Abschnitts vom zweiten Abschnitt im Bereich der Einlassöffnung gewährleistet, so dass ein durch die Einlassöffnung fließender Saugstrom nicht dazu führt, dass sich eine Oberfläche des zweiten Abschnitts an die Oberfläche des ersten Abschnitts saugt und somit den Saugstrom unterbricht. Dies ist insbesondere dann vorteilhaft, wenn es sich bei dem zweiten Abschnitt um einen Abschnitt aus flexiblem Material wie beispielsweise einen Abströmschlauch handelt.

In einer weiteren Ausführungsform weist der zweite Abschnitt eine die weitere Einlassvorrichtung umfassende bzw. die weitere Einlassöffnung teilweise umfassende Hülse auf. Diese kann als zusätzliches Spezialteil beispielsweise mit einem flexiblen Bereich des zweiten Abschnitts verbunden werden. Dabei ist es vorteilhaft, wenn die Hülse im Arbeitszustand, welcher beispielsweise durch Entfaltung der Pumpenanordnung an ihrem Arbeitsort im Körper definiert ist, formfest ist und somit gegenüber einem Fluid einen geeigneten Widerstand bildet, so dass der Saugstrom durch die Hülse kanalisiert wird und in den Strömungskanal einströmt.

Anstelle einer Hülse kann auch ein weiterer Schlauchabschnitt oder ein Rohr verwendet werden.

In einer weiteren Ausführungsform ist die Pumpe eine komprimierbare Pumpe, was zu einem leichteren Einführen der Pumpe in die Blutbahn oder ein Gefäß führt.

Weiterhin ist es vorteilhaft, wenn die Pumpe eine Axialpumpe ist, welche auf einer rotierbaren Welle, die die Pumpe antreibt, befestigt ist.

Nachfolgend soll die Erfindung anhand einiger Ausführungsbeispiele genauer erläutert werden. Es zeigen:
- Fig. 1: die Verwendung einer Pumpenanordnung in einem Herzen;
- Fig. 2: eine schematische Darstellung einer Ausführungsform der Pumpenanordnung;
- Fig. 3: eine schematische Darstellung einer Einlassöffnung einer Ausführungsform der Pumpenanordnung;
- Fig. 4: eine Ausführungsform einer Pumpenanordnung;
- Fig. 5a: eine weitere Ausführungsform einer Pumpenanordnung;
- Fig. 5b: eine schematische Darstellung der Pumpenanordnung der Fig. 5a;
- Fig. 6: eine weitere Ausführungsform einer Pumpenanordnung;
- Fig. 7: eine weitere Ausführungsform einer Pumpenanordnung;
- Fign. 8a-8c: Querschnitte durch verschiedene Pumpenanordnungen.

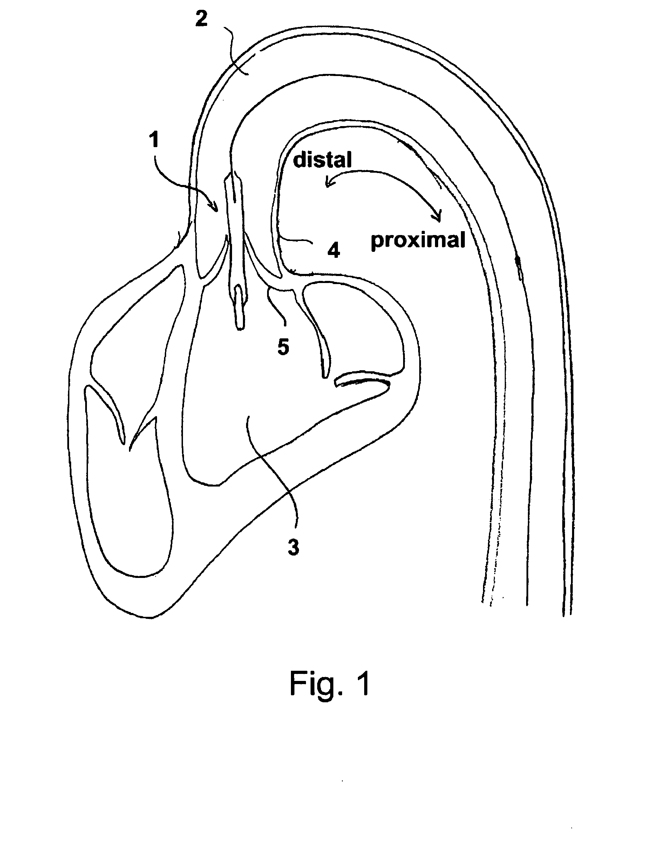

In der Fig. 1 ist eine mögliche Verwendung für die Pumpenanordnung 1 dargestellt. Die Pumpenanordnung 1 umfasst einen durch das Blutgefäß 2 reichenden länglichen Katheter, in welchem eine Welle verläuft, welche die in der Pumpenanordnung 1 vorhandene, als Rotor ausgebildete Pumpe antreibt. Das proximale Ende der Pumpenanordnung (ohne Katheter betrachtet) befindet sich im Blutgefäß 2, wohingegen das distale, die Pumpe umfassende Ende der Pumpenanordnung 1 sich in der Herzkammer 3 befindet. Das Blutgefäß 2 wird durch die Gefäßwand 4 begrenzt. Weiterhin begrenzt die Klappe 5, welche sich rhythmisch öffnet und schließt, die Herzkammer 3 und ermöglicht den Blutfluss von der Herzkammer 3 in das Blutgefäß 2.

Neben der dargestellten Verwendung einer erfindungsgemäßen Pumpenanordnung sind weitere Verwendungen möglich. So kann die Pumpe beispielsweise in einem anderen körpereigenen Gefäß zur Erhöhung der Förderleistung eingesetzt werden.

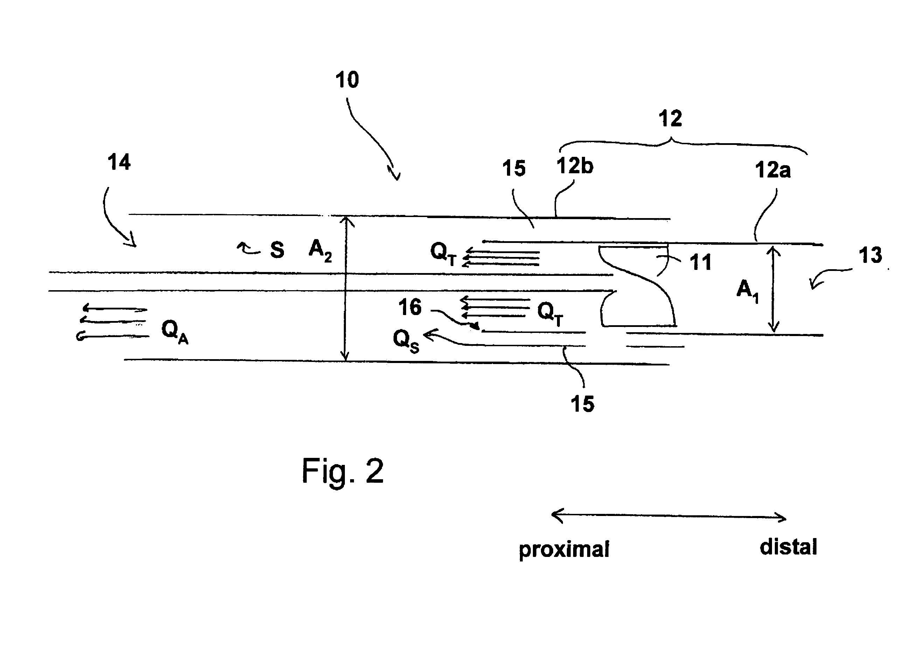

Anhand der Fig. 2 soll die Wirkungsweise einer erfindungsgemäßen Pumpenanordnung erläutert werden. Die Pumpenanordnung 10 umfasst eine Pumpe 11, welche als Rotor ausgebildet ist. Die Pumpe 11 wird mittels einer dargestellten, jedoch nicht bezeichneten Welle in Drehung versetzt und vermag so einen Treibstrom Q_{T} zu transportieren. Die Pumpenanordnung 10 weist eine Umhüllung 12 auf, welche einen ersten Abschnitt 12a und einen zweiten Abschnitt 12b umfasst. In dem ersten Abschnitt 12a befindet sich eine Ansaugöffnung 13, durch welche ein Fluid in das Lumen des ersten Abschnitts 12a eintreten kann, durch die Pumpe 11 angesogen und als Treibstrom Q_{T} in Richtung der Ausströmöffnung 14 transportiert wird. Die Umhüllung 12 definiert zwischen der Ansaugöffnung 13 und der Ausströmöffnung 14 den Strömungskanal S, welcher beim Ausführungsbeispiel der Fig. 2 das Lumen des ersten Abschnitts 12a vollständig und ein Lumen des zweiten Abschnitts 12b teilweise umfasst.

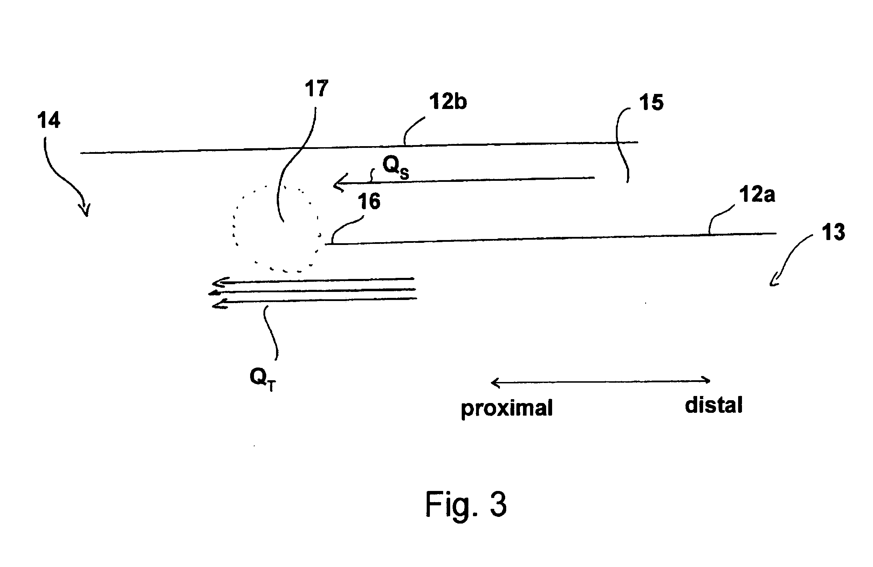

Zwischen dem proximalen Ende des ersten Abschnitts 12a und dem distalen Ende des zweiten Abschnitts 12b überlappen sich der erste und zweite Abschnitt. Durch die Überlappung wird eine Einlassöffnung 15 definiert, durch welche das Fluid von einem Bereich außerhalb des Lumens des ersten Abschnitts 12a in den Strömungskanal S eintreten kann. Aufgrund des durch die Pumpe geförderten Treibstroms Q_{T} kommt es im Bereich des proximalen Endes des ersten Abschnitts 16 zu einem Druckabfall im Bereich 17. Dies ist in der Fig. 3 dargestellt.

Durch den Druckabfall im Bereich 17 wird durch die Einlassöffnung 15 weiteres Fluid in Richtung der Ausströmöffnung 14 gesogen, welches als Saugstrom Q_{S} proximal des proximalen Endes des ersten Abschnitts 16 in den Strömungskanal eintritt. Somit mündet die Eingangsöffnung 15 in den Strömungskanal S.

Der erste Abschnitt 12a und der zweite Abschnitt 12b umfassen beide ein Lumen. Dabei weist das Lumen des ersten Abschnitts 12a eine Querschnittsfläche A₁ auf, das Lumen des zweiten Abschnitts 12b eine Querschnittsfläche A₂. Im vorliegenden Ausführungsbeispiel bleiben die Querschnitte A₁ und A₂ über die gesamte Länge des jeweiligen Abschnitts gleich; dies ist jedoch kein zwingendes Merkmal. Durch den zwischen dem distalen Ende des zweiten Abschnitts 12b und dem proximalen Ende des ersten Abschnitts 12a parallel zum Treibstrom verlaufenden, als Einlassöffnung 15 ausgebildeten Kanal bekommt der Saugstrom bereits eine Impulsrichtung in Richtung der Ausströmöffnung 14. Durch den zusätzlichen Saugstrom Q_{S} ist das an der Ausströmöffnung 14 ausgeströmte Volumen pro Zeit Q_{A} größer als der die Pumpe passierende Treibstrom Q_{T}.

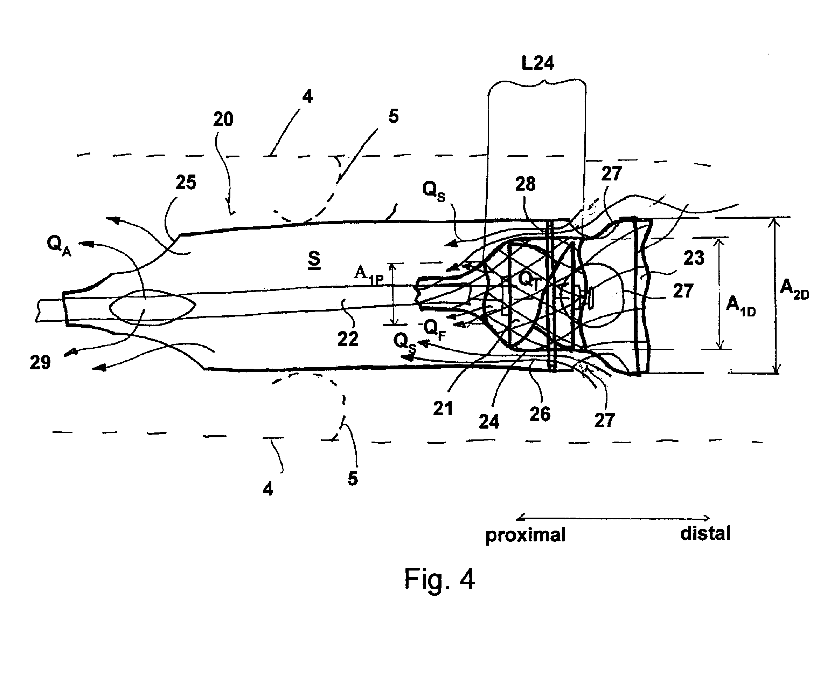

Eine weitere Ausführungsform einer Pumpenanordnung ist in der Fig. 4 beschrieben. Die Pumpenanordnung 20 befindet sich in einem Blutgefäß, welches durch die Gefäßwände 4 begrenzt wird. Das distale Ende der Pumpenanordnung 20 befindet sich distal der Klappe 5, das proximale Ende befindet sich proximal der Klappe 5.

Die Pumpenanordnung 20 umfasst einen komprimierbaren Rotor 21, welcher auf der Welle 22 einseitig befestigt ist. Die Lagerung befindet sich am proximalen Ende des Rotors. Der Rotor 21 wird durch ein Gehäuse 23 umgeben, welches aus Nitinol hergestellt sein kann. Das Gehäuse besteht aus einzelnen Nitinolfäden, -drähten oder -streben, welche sich gegenseitig kreuzen und ein Rautenmuster erzeugen. Das Fluid kann durch die Rauten hindurchtreten und so den Rotor 21 erreichen.

Teilweise ist das Gehäuse 23 mit einer Ummantelung 24 fluiddicht abgedeckt. Dabei erstreckt sich die Ummantelung 24 über eine Länge L₂₄, so dass ein durch den Rotor angetriebener Treibstrom Q_{T} gebündelt wird und am proximalen Ende der Ummantelung 24 aus dem Gehäuse 23 austritt und in Richtung der Ausströmöffnungen 29 fließt, welche in einem Abströmschlauch 25 angeordnet sind.

Die Ummantelung 24 bildet bei der Ausführung der Pumpenanordnung 20 den ersten Abschnitt der Umhüllung, der Abströmschlauch 25 bildet den zweiten Abschnitt der Umhüllung. Das distale Ende des Abströmschlauchs ist am Gehäuse 23 befestigt und liegt distaler als das proximale Ende der Umhüllung 24.

Die Umhüllung 24 verjüngt sich vom Bereich des Rotors 21 in proximaler Richtung. So weist das durch die Umhüllung 24 gebildete Lumen im Bereich des Rotors 21 eine Querschnittsfläche A_{1D} auf, welche größer ist als die Querschnittsfläche A_{1P} des proximalen Endes der Umhüllung 24. Hierdurch wird ein Düseneffekt erzeugt, welcher den Treibstrom Q_{T} nach dem Prinzip des Venturirohrs beschleunigt, so dass dieser am proximalen Ende der Umhüllung 24 mit einer höheren Flussgeschwindigkeit in Richtung der Ausströmöffnungen 29 strömt. Zwischen der Umhüllung 24 und dem Abströmschlauch 25 befindet sich der Ansaugkanal 26, welcher durch die Eingänge 27 zugänglich ist. Aus der Fig. 4 ist erkennbar, dass mehrere Eingänge 27 vorhanden sind, wobei die Eingänge als kreisrunde Ausschnitte des Abströmschlauchs im Bereich seines distalen Endes ausgeführt sind. Aufgrund des im Bereich des austretenden Treibstroms Q_{T} verringerten Drucks wird ein Saugstrom Q_{S} durch den Eingang 27 und den Ansaugkanal 26 angesogen und mündet in den Strömungskanal S, welcher den gesamten Förderstrom zu den Ausströmöffnungen 29 transportiert.

Proximal der Eingänge 27 und den Abströmschlauch 25 radial umlaufend befindet sich ein Stützring 28, welcher im Arbeitszustand der Pumpe formstabil ist. So wird ein Ansaugen der Oberfläche des Abströmschlauchs 25 an die Umhüllung 24 aufgrund des auftretenden Saugstroms verhindert. Der Ansaugkanal 26 bleibt somit geöffnet, und es wird weiteres Fluid durch den Ansaugkanal 26, bewirkt durch den Treibstrom Q_{T}, in den Strömungskanal S gesogen.

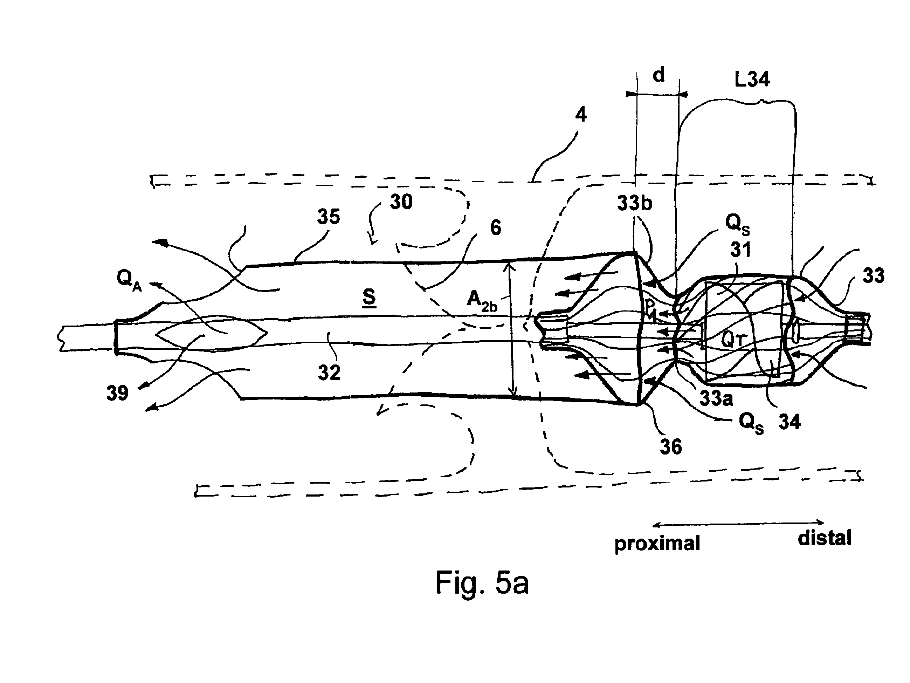

Eine weitere Ausführungsform der erfindungsgemäßen Pumpanordnung ist in der Fig. 5a dargestellt. Die Pumpanordnung 30 umfasst einen Rotor 31, welcher auf einer Achse 32 beidseitig, d. h. distal und proximal, gelagert ist. Der Rotor 31 ist in einem Gehäuse 33 angeordnet, welches abschnittsweise durch eine PU-Beschichtung 34 ummantelt ist. Die PU-Beschichtung 34 erstreckt sich dabei über eine Länge L₃₄ bis zu einem proximal zum proximalen Ende des Rotors 31 liegenden Bereich hin. Das Gehäuse 33 weist eine Einschnürung 33a auf und weitet sich proximal der Einschnürung 33a zu einer Ausbauchung 33b auf. Im Bereich der Ausbauchung 33b ist der Abströmschlauch 35 mit dem Gehäuse 33 stoffschlüssig verbunden. Die Ausbauchung 33b und die Einschnürung 33a sind entlang der Achse 32 gemessen um einen Abstand d voneinander beabstandet, der etwa 0 bis 1/4 des Durchmessers der Einschnürung 33a beträgt. Dabei ist der Abstand d derart gewählt, dass aufgrund des aus dem proximalen Ende der PU-Beschichtung 34 austretenden, durch den Rotor 31 getriebenen Treibstroms Q_{T} ein Saugstrom Q_{S} durch die sich zwischen der PU-Beschichtung 34 und dem Abströmschlauch 35 ergebende Eingangsöffnung 36 nachgesogen wird. Der aus der Ummantelung austretende Treibstrom Q_{T} wird mit einem Druck P₁ ausgeströmt. Außerhalb der Ummantelung 34 liegt ein Druck P₂ an, welcher geringer ist als der Druck P₁. Durch diesen Druckunterschied wird ein Saugstrom Q_{S} in die Eingangsöffnung 36 eingesogen und durch den Abströmschlauch zu der Ausströmöffnung 39 hin transportiert, wo dieser unter einem Druck P₃, welcher größer als der Druck P₂ ist, als Gesamtstrom Q_{A} ausgestoßen wird. Der Gesamtstrom Q_{A} ist dabei größer als der Treibstrom Q_{T}.

Auch wenn der Strömungskanal S, welcher sich zwischen der Ansaugöffnung distal des Rotors 31 und der Ausströmöffnung 39 erstreckt, zwischen dem proximalen Ende der PU-Beschichtung 34 und dem distalen Ende des Abströmschlauchs 35 fluiddurchlässig ist, mündet die Einströmöffnung 36 dennoch in den Strömungskanal, welcher durch den Flussverlauf des Treibstroms definiert ist. Ist der Treibstrom entsprechend hoch, tritt dieser nahezu direkt in den Abströmschlauch ein.

Durch die zusätzlich zur distal des Rotors 31 gelegenen Ansaugöffnung vorhandene Eingangsöffnung ist es möglich, dass ein Teilstrom des an der Ausströmöffnung 39 austretenden Gesamtstroms Q_{A} den Rotor 31 nicht passiert und somit keine Gefahr der Blutschädigung durch den Rotor 31 besteht.

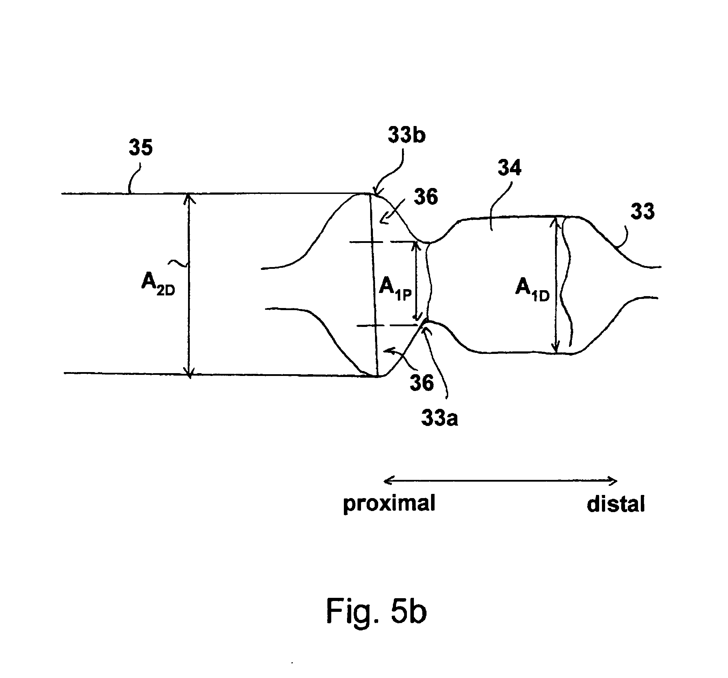

Die Ausführungsform der Pumpenanordnung 30 der Fig. 5a ist in der Fig. 5b nochmals schematisch dargestellt. Hier ist erkennbar, dass das distale Ende der PU-Beschichtung 34 eine Querschnittsfläche A_{1D} aufweist, welche größer gegenüber der Querschnittsfläche A_{1P} ist, welche am proximalen Ende der PU-Beschichtung 34 vorliegt. Somit verjüngt sich das durch die PU-Beschichtung 34 umschlossene Lumen, was eine Wirkungsgradverbesserung zur Folge hat. Die Querschnittsfläche A_{2D} des Lumens des Abströmschlauchs 35 ist wiederum größer als die Querschnittsfläche A_{1P}. Somit wird zumindest durch den Bereich der Querschnittsfläche A_{2D}, welcher nach der Subtraktion der Querschnittsfläche A_{1P} verbleibt, eine Eingangsöffnung 36 definiert. Diese mündet wiederum in den Strömungskanal S.

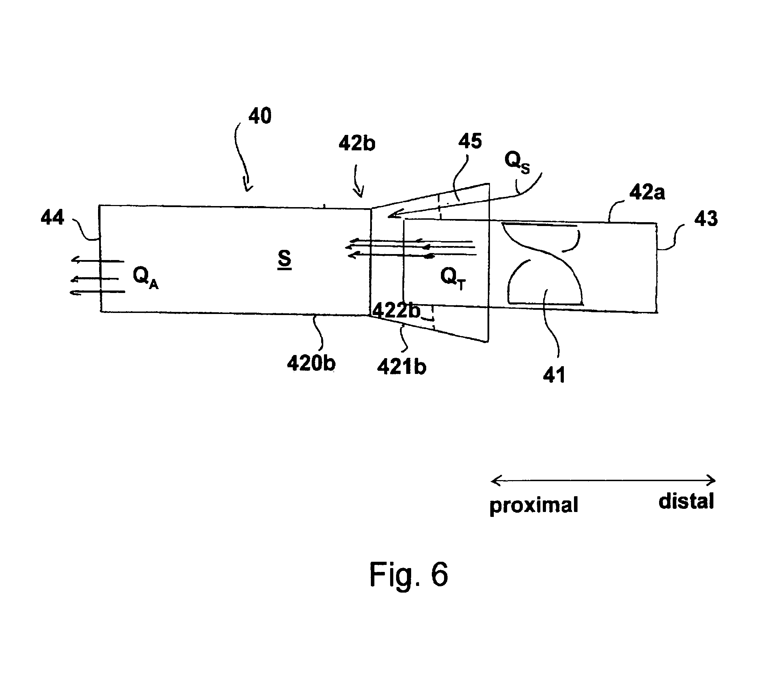

Eine weitere Ausführungsform einer Pumpenanordnung ist in der Fig. 6 dargestellt. Hierbei wird auf eine detaillierte Beschreibung der Achse und des Pumpenantriebs verzichtet. Die Pumpenanordnung 40 umfasst einen Rotor 41 sowie einen ersten Abschnitt 42a und einen zweiten Abschnitt 42b einer Umhüllung. Am distalen Ende des ersten Abschnitts 42a befindet sich die Ansaugöffnung 43, welche der Pumpe 41 Fluid zuführt. Das der Pumpe 41 zugeführte Fluid wird beschleunigt und als Treibstrom Q_{T} am proximalen Ende des ersten Abschnitts 42a ausgeworfen. Der zweite Abschnitt 42b setzt sich aus einem flexiblen Bereich 420b zusammen, welcher mit einer komprimierbaren, im Arbeitszustand des Rotors starren, formstabilen Hülse 421b verbunden ist. Die komprimierbare Hülse 421b ist mittels Kunststofffäden oder Drähten 422b mit dem ersten Abschnitt 42a verbunden. Der vom distalen Ende der Hülse 421b zum proximalen Ende der Hülse 421b verjüngend verlaufende Querschnitt bewirkt in Verbindung mit dem Treibstrom Q_{T} ein Ansaugen eines Saugstroms Q_{S} durch die Eingangsöffnung 45, welche zwischen der Hülse 421b und dem ersten Abschnitt 42a gebildet wird, wobei sich der Saugstrom Q_{S} mit dem Treibstrom Q_{T} vereint und im Strömungskanal S als Gesamtstrom Q_{A} aus der Ausströmöffnung 44 ausströmt. Aus der Fig. 6 ist wiederum offensichtlich, dass die Eingangsöffnung 45 in den Strömungskanal S mündet.

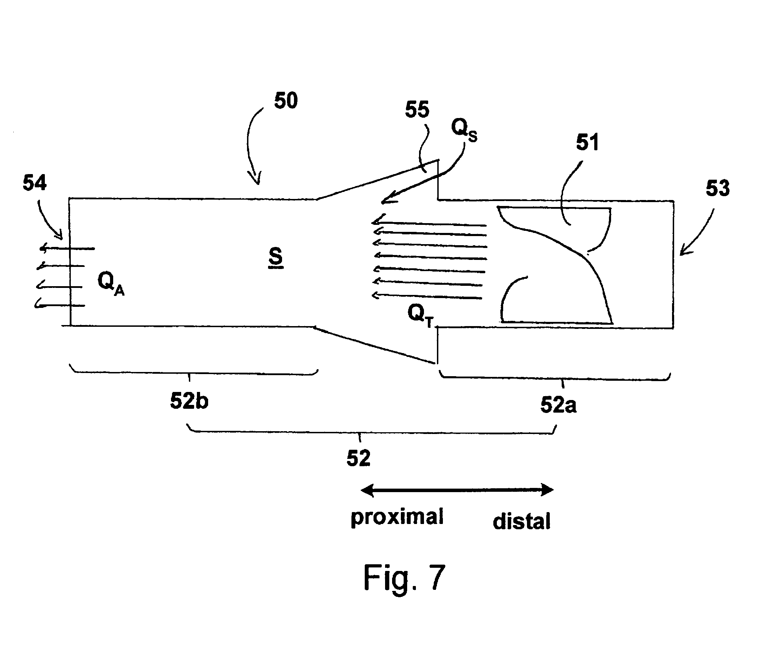

Eine weitere Ausführungsform einer Pumpenanordnung ist in der Fig. 7 dargestellt. Die Pumpenanordnung 50 umfasst eine Pumpe 51, welche als Axialpumpe mit einem Rotor ausgebildet ist. Des Weiteren ist eine Umhüllung 52 vorhanden, welche in einen ersten Abschnitt 52a und einen zweiten Abschnitt 52b unterteilbar ist. Dabei sind der erste und der zweite Abschnitt stoffschlüssig miteinander verbunden bzw. einstückig hergestellt. Am distalen Ende der Umhüllung 52 befindet sich eine Ansaugöffnung 53, welche dem Rotor Fluid zuführt, so dass im Arbeitszustand des Rotors ein Treibstrom Q_{T} gefördert wird. Der Treibstrom Q_{T} wird in Richtung der Ausströmöffnung 54 befördert. Zwischen dem ersten Abschnitt 52a und dem zweiten Abschnitt 52b befindet sich eine Eingangsöffnung 55, durch welche ein durch den Treibstrom Q_{T} bewirkter Saugstrom Q_{S} in den durch die Umhüllung 52 definierten Strömungskanal S eintreten kann. Die Besonderheit an dieser Ausführungsform ist, dass die Umhüllung 52 einstückig ausgeführt ist, im Gegensatz zu den bislang dargestellten Ausführungsformen, bei welchen der erste Abschnitt ein eigenes Bauteil gegenüber dem zweiten Abschnitt darstellt.

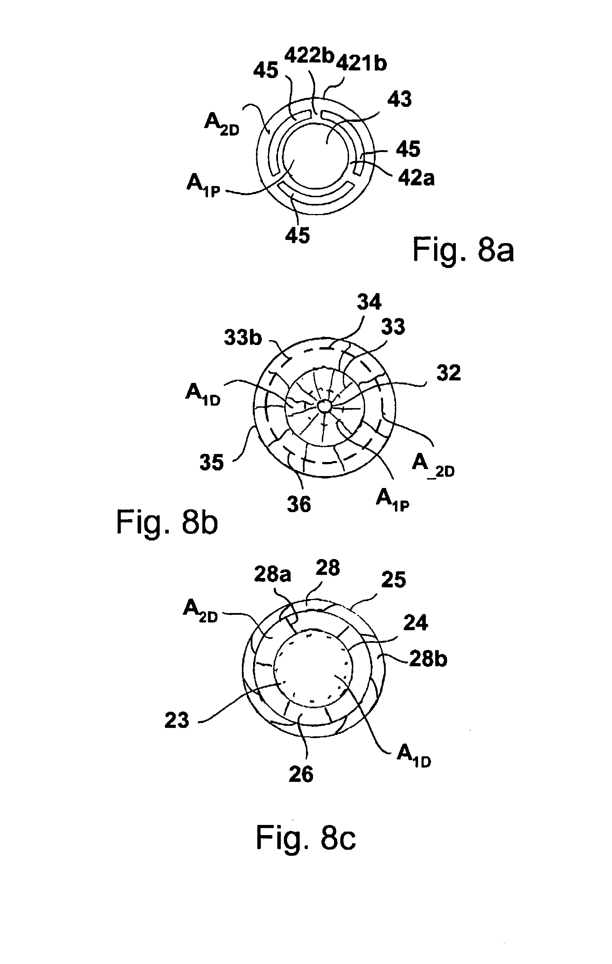

Anhand der Fign. 8a-c sollen einige verschiedene Geometrien von Eingängen der Einlassöffnungen dargestellt werden.

In der Fig. 8a ist ein Querschnitt des Ausführungsbeispiels der Fig. 6 dargestellt. Zu sehen ist die Ansaugöffnung 43 mit einer Querschnittsfläche A_{1P}. Proximal, d. h. weiter in die Bildebene hinein, davon gelegen befindet sich die Hülse 421b mit dem an ihrem weitesten Umfang gemessenen Querschnitt A_{2D}. Die Kunststofffäden 422b verbinden die Hülse 421b mit dem ersten Abschnitt 42a.

In der Fig. 8b ist das Ausführungsbeispiel der Fig. 5a dargestellt. Es ist die Ansaugöffnung 33 erkennbar, welche durch die PU-Beschichtung 34 definiert wird. Die PU-Beschichtung 34 definiert zugleich ein Lumen, welches im Bereich des Rotors eine Querschnittsfläche A_{1D} aufweist. In der Mitte der Ansaugöffnung 33 ist zudem die Achse 32 erkennbar. Proximal zum Rotor (vgl. Fig. 5a) verjüngt sich das Gehäuse 33, welches durch Nitinolfäden, -drähte oder -streben gebildet wird, zu einer Querschnittsfläche A_{1P}, welche durch die Einschnürung 33a definiert wird. Proximal gelegen weitet sich das Gehäuse 33 zu einer Ausbauchung 33b auf, wobei im Bereich der Ausbauchung der Ausströmschlauch 35 mit dem Gehäuse verbunden wird. Anhand der in der Fig. 8b gezeigten Darstellung ist deutlich erkennbar, dass der zwischen dem Abströmschlauch 35 und der PU-Beschichtung 34 liegende Bereich 36 als Eingangsöffnung für einen Saugstrom dient.

In der Fig. 8c ist das Ausführungsbeispiel der Fig. 4 dargestellt. Hierbei ist der Querschnitt auf Höhe des Stützrings 28 gezeigt. Zu erkennen ist das durch die Ummantelung 24 des ersten Abschnitts definierte Lumen mit der Querschnittsfläche A_{1D}. Zwischen der Ummantelung 24 und dem Abströmschlauch 25 befindet sich der Ansaugkanal 26, durch welchen zusätzliches Fluid, getrieben durch den durch das Lumen der Ummantelung 24 in proximaler Richtung laufenden Treibstrom, angesogen wird. Der Abströmschlauch 25 weist in diesem Bereich eine Querschnittsfläche von A_{2D} auf. Der Stützring 28 ist deutlich zu erkennen, ebenso die Verbindungsstreben 28a, welche den Stützring mit der Ummantelung 24 verbinden. Der Stützring ist aus mehreren Segmenten 28b aufgebaut, welche zum Einführen der Pumpenanordnung mithilfe eines Katheters in einen gefalteten Zustand bringbar sind.

### Bezugszeichenliste

- 1, 10, 20, 30, 40, 50: Pumpenanordnung
- 2: Blutgefäß
- 3: Herzkammer
- 4: Gefäßwand
- 5: Herzklappe
- 6: Gefäßklappe
- 11, 41, 51: Pumpe
- 12, 42, 52: Umhüllung
- 12a, 42a, 52a: 1. Abschnitt der Umhüllung
- 12b, 42b, 52b: 2. Abschnitt der Umhüllung
- 13, 43, 53: Ansaugöffnung
- 14, 44, 54: Ausströmöffnung
- 15: Einlassöffnung
- 16: proximales Ende des 1. Abschnitts
- 17: Bereich Druckabfall
- 21, 31: Rotor
- 22, 32: Achse
- 23, 33: Gehäuse
- 24, 34: Ummantelung
- L24, L34: Länge der Ummantelung
- 25, 35, 420b: Abströmschlauch
- 26, 36: Ansaugkanal
- 27: Eingänge
- 28: Stützring/Abstandshalter
- 33a: Einschnürung
- 33b: Ausbauchung
- 421b: Hülse
- 422b: Abstandshalter
- Q_{T}: Förderstrom
- Q_{S}: Saugstrom
- Q_{A}: Gesamtstrom
- A₁, A₂, A_{1D}, A_{1P}, A_{2D}: Querschnitt

## Patentansprüche

1. Pumpenanordnung (1, 10, 20, 30, 40, 50), insbesondere zum Einsatz in körpereigenen Gefäßen, mit einer Pumpe (11, 41, 51) und einer die Pumpe aufnehmenden und einen Strömungskanal (S) begrenzenden Umhüllung (12, 42, 52) mit einer distalen Ansaugöffnung (13, 43, 53) und einer proximalen Ausströmöffnung (14, 29, 39, 44, 54) zur Erzeugung eines Treibstroms (Q_{T}) mittels der Pumpe, wobei die Pumpe in einem ersten fluiddichten und die distale Ansaugöffnung aufweisenden Abschnitt (12a, 42a, 52a) angeordnet ist und ein zweiter fluiddichter Abschnitt (12b, 42b, 52b) die proximale Ausströmöffnung umfasst, **dadurch gekennzeichnet, dass** zwischen dem ersten und dem zweiten Abschnitt eine weitere Einlassöffnung (15) vorhanden ist, wobei der erste Abschnitt und der zweite Abschnitt derart zueinander angeordnet sind, dass die Einlassöffnung proximal der Pumpe in den Strömungskanal (S) mündet.

2. Pumpenanordnung nach Anspruch 1, **dadurch gekennzeichnet, dass** der Querschnitt (A_{1P}) des proximalen Endes des ersten Abschnitts kleiner als der Querschnitt (A_{2P}) des distalen Endes des zweiten Abschnitts ist.

3. Pumpenanordnung nach einem der vorhergehenden Ansprüche, **dadurch gekennzeichnet, dass** sich der Querschnitt (A_{1D}, A_{1P}) des ersten Abschnitts zu seinem proximalen Ende hin verjüngt.

4. Pumpenanordnung nach einem der vorhergehenden Ansprüche, **dadurch gekennzeichnet, dass** das distale Ende des zweiten Abschnitts distaler als das proximale Ende des ersten Abschnitts angeordnet ist und die Einlassöffnung als zwischen dem ersten und zweiten Abschnitt verlaufender Ansaugkanal (26, 36) ausgebildet ist.

5. Pumpenanordnung nach einem der vorhergehenden Ansprüche, **dadurch gekennzeichnet, dass** das distale Ende des zweiten Abschnitts proximaler als oder auf gleicher Höhe wie das proximale Ende des ersten Abschnitts angeordnet ist.

6. Pumpenanordnung nach einem der vorhergehenden Ansprüche, **dadurch gekennzeichnet, dass** der zweite Abschnitt (12b, 42b, 52b) einen Bereich aus einem flexiblen Material umfasst.

7. Pumpenanordnung nach einem der vorhergehenden Ansprüche, **dadurch gekennzeichnet, dass** ein die Pumpe aufnehmendes Gehäuse (23, 33) vorhanden ist.

8. Pumpenanordnung nach Anspruch 7, **dadurch gekennzeichnet, dass** der erste Abschnitt eine Ummantelung (24, 34) des Gehäuses (23, 33) ist.

9. Pumpenanordnung nach einem der Ansprüche 7 oder 8, **dadurch gekennzeichnet, dass** der zweite Abschnitt mit dem Gehäuse (23, 33) verbunden ist.

10. Pumpenanordnung nach einem der vorhergehenden Ansprüche, **dadurch gekennzeichnet, dass** der erste und der zweite Abschnitt stoffschlüssig miteinander verbunden sind.

11. Pumpenanordnung nach einem der vorhergehenden Ansprüche, **dadurch gekennzeichnet, dass** der zweite Abschnitt einen Abströmschlauch (25, 35, 420b) umfasst.

12. Pumpenanordnung nach einem der vorhergehenden Ansprüche, **dadurch gekennzeichnet, dass** der zweite Abschnitt im Bereich der Einlassöffnung einen Stützring (28) aufweist.

13. Pumpenanordnung nach einem der vorhergehenden Ansprüche, **dadurch gekennzeichnet, dass** der zweite Abschnitt eine die weitere Einlassöffnung (15) umfassende Hülse (421b) umfasst.

14. Pumpenanordnung nach einem der vorhergehenden Ansprüche, **dadurch gekennzeichnet, dass** die Pumpe (11) eine komprimierbare Pumpe ist.

15. Pumpenanordnung nach einem der vorhergehenden Ansprüche, **dadurch gekennzeichnet, dass** eine rotierbare Welle (16, 22, 32) vorhanden ist, auf welcher die Pumpe (11) angeordnet ist.
